# EUROPÄISCHE PATENTANMELDUNG

(11) **EP 4 335 466 A1**
(43) Veröffentlichungstag der Anmeldung: **13.03.2024**
(21) Anmeldenummer: 22194533.0
(22) Anmeldetag: 08.09.2022
(51) Int. Cl.: A61L 27/10, A61L 27/12, A61L 27/44, A61L 27/46, A61L 27/50, A61L 27/54

(54) **ZUSAMMENSETZUNG ZUR HERSTELLUNG EINES KNOCHENERSATZMATERIALS, VERFAHREN ZUR HERSTELLUNG EINES PHARMAZEUTISCHEN WIRKSTOFFTRÄGERS, PHARMAZEUTISCHER WIRKSTOFFTRÄGER SOWIE DESSEN VERWENDUNG**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Die Erfindung betrifft eine Zusammensetzung zur Herstellung eines Knochenersatzmaterials, welche mit Wasser oder einer wässrigen Lösung in einer zementartigen Abbindereaktion reagiert und einen Festkörper ausbildet.

Die Erfindung betrifft weiterhin ein Verfahren zur Herstellung eines pharmazeutischen Wirkstoffträgers mit einer derartigen Zusammensetzung, den derartig hergestellten pharmazeutischen Wirkstoffträger sowie die Verwendung einer derartigen Zusammensetzung oder eines derartigen pharmazeutischen Wirkstoffträgers zur lokalen Freisetzung mindestens eines pharmazeutischen Wirkstoffs.

## Beschreibung

Die Erfindung betrifft eine Zusammensetzung zur Herstellung eines Knochenersatzmaterials, welche mit Wasser oder einer wässrigen Lösung in einer zementartigen Abbindereaktion reagiert und einen Festkörper ausbildet.

Die Erfindung betrifft weiterhin ein Verfahren zur Herstellung eines pharmazeutischen Wirkstoffträgers mit einer derartigen Zusammensetzung, den derartig hergestellten pharmazeutischen Wirkstoffträger sowie die Verwendung einer derartigen Zusammensetzung oder eines derartigen pharmazeutischen Wirkstoffträgers zur lokalen Freisetzung mindestens eines pharmazeutischen Wirkstoffs.

### Hintergrund der Erfindung

Mit Wasser oder einer wässrigen Lösung in einer zementartigen Abbindereaktion unter Ausbildung eines Festkörpers reagierende Zusammensetzungen, sogenannte mineralische Reaktivsysteme, wie beispielsweise Zemente und Gipse, kommen seit langer Zeit für eine Vielzahl von Anwendung zum Einsatz. Typischerweise bestehen sie aus mineralischen Pulvern, die mit Wasser angemischt werden und innerhalb von wenigen Minuten bis gar Monaten ihre endgültige Zusammensetzung und somit Festigkeit erreichen. Die Abbindereaktion besteht üblicherweise in einer Lösung von wasserlöslichen Pulverkomponenten und anschließender Ausfällung einer stabileren oder schwerer löslichen Phase oder Salzform, bzw. in einer Umkristallisation von metastabilen Pulverkomponenten zu einer unter den Anwendungsbedingungen thermodynamisch stabilen Modifikation. Häufig reagieren auch zwei oder mehr wasserlösliche Pulverbestandteile zu einer schwerlöslichen Substanz.

Oftmals werden den Zusammensetzungen Anteile an anorganischen Füllstoffen zugesetzt, welche Einfluss auf die Abbindereaktion und/oder die Festigkeit des ausgebildeten Festkörpers haben können.

Derartige Zusammensetzungen kommen ebenfalls seit einigen Jahren vermehrt in medizinischen Anwendungen, wie beispielsweise dentalen Füllungen (z. B. Wurzelfüllungen) oder auch als Knochenersatzmaterialien, wie beispielsweise in der Mund-, Kiefer- und Gesichtschirurgie und der Orthopädie, zum Einsatz. Insbesondere in der Orthopädie werden hohe Anforderungen an die Reinheit, Bioverträglichkeit als auch das Abbindeverhalten der Zusammensetzungen, insbesondere deren Volumenstabilität beim Abbinden sowie der Geschwindigkeit der Abbindereaktion, gestellt.

Insbesondere die Verwendung von Calciumsulfat zum Auffüllen von Knochenkavitäten ist seit 1892 durch die Arbeiten von Dreesmann bekannt (H. Dressman: Ueber Knochenplombierung. Beitr. Klin. Chir. 9 (1892) 804-810.). Auf Basis von Calciumsulfat-Hemihydrat wurden eine Vielzahl von selbstaushärtenden Knochenersatzmaterialien vorgeschlagen. Diese basieren auf pulverförmigen Calciumsulfat-Hemihydrat, das mit Wasser oder wässrigen Lösungen vermischt wird, wobei eine Paste entsteht, welche unter Ausbildung von Calciumsulfat-Dihydrat innerhalb weniger Minuten aushärtet. Weiterhin sind eine Vielzahl von anorganischen Knochenersatzmaterialien bekannt, bei denen metastabile Calciumphosphate nach Vermischen mit Wasser oder wässrigen Lösungen in Gegenwart geeigneter Beschleuniger aushärten. Exemplarisch seien für derartige Zusammensetzungen zur Herstellung von Knochenersatzmaterialien, welche durch Vermischung von Calciumsulfaten und/oder Calciumphosphaten mit Wasser oder wässrigen Lösungen aushärten, folgende Patentschriften genannt: EP 1 301 219 B1, EP 1 601 387 B1, EP 1 948 255 B1, EP 2 988 789 B1, EP 3 157 862 B1 und EP 3 166 651 B1.

EP 2 170 245 B1 beschreibt ein Knochenersatzmaterial auf Basis eines hydraulischen Zements, wobei dessen partikulären Bestandteile mit einer organischen Trägerflüssigkeit unter Bereitstellung einer Paste, Suspension oder Dispersion vermischt werden. Die Trägerflüssigkeit hat dabei eine Wasseraufnahme von kleiner 25 Volumen-%, ist also im weitestgehenden Sinne nicht mit Wasser löslich, und bildet mit Wasser Emulsionen. Vorzugsweise enthält die Zusammensetzung Tenside.

Als nachteilig gestaltet sich bei derartigen, nicht in Wasser löslichen Trägerflüssigkeiten, dass das Wasser oder die wässrige Lösung, welche zum Aushärten in die Zusammensetzung aufgenommen wird, zu einer Zunahme des ursprünglichen Volumens der Zusammensetzung führt, da die Trägerflüssigkeit nicht vollständig durch das Wasser oder die wässrige Lösung ersetzt wird. Diese Volumenzunahme ist insbesondere beim Befüllen von Kavitäten in Knochen nachteilig, da sie zu einer Schädigung des umliegenden Knochengewebes führen kann. Weiterhin kommt es bei derartigen Trägerflüssigkeiten zur Ausbildung von Poren, welche die Festigkeit des Knochenersatzmaterials negativ beeinträchtigen.

Die Volumenzunahme kann zwar durch den Einsatz von Tensiden verringert werden, allerdings sollte der Einsatz von Tensiden im menschlichen Körper aufgrund deren potenziell membranschädigender Wirkung vermieden werden.

Weiterhin werden als Beimischungen zu den nicht wasserlöslichen Bestandteilen der Trägerflüssigkeiten oligomere oder polymere Derivate des Ethylenglykols und des Propylenglykols vorgeschlagen, welche zumindest teilweise wasserlöslich sind.

WO 2002/062721 A1 und WO 2004/093734 A2 offenbaren Zusammensetzung basierend auf Calciumphosphaten mit einer Trägerflüssigkeit aus Glycerin, die mit Wasser oder wässrigen Lösungen zu einem Festkörper aushärten können. Glycerin umgeht zwar die Problematik einer Volumenzunahme nicht wasserlöslicher Trägerflüssigkeiten beim Aushärten, allerdings können die allgemeinen Nachteile Glycerin-basierter Trägerflüssigkeiten, insbesondere eine verringerte Lagerstabilität, auch durch den offenbarten Zusatz von Säuren und Gelbildnern nicht gelöst werden.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, Zusammensetzungen bereitzustellen, welche mit Wasser oder wässrigen Lösungen in einer zementartigen Abbindereaktion zu Festkörpern abbinden, welche beim Abbinden im Wesentlichen keine Volumenzunahme erfahren und möglichst lange lagerstabil sein sollen. Die Zusammensetzungen sollen als Knochenersatzmaterial verwendbar sein. Die Zusammensetzungen sollen von einem Anwender plastisch in beliebige Form gebracht werden können. Die Zusammensetzungen sollen ohne Zeitdruck in Kavitäten, insbesondere Knochenkavitäten, eingebracht werden können und dort aufgrund deren Viskosität bis zur deren Abbindung verbleiben. Weiterhin sollen die Zusammensetzungen durch Kontakt der Zusammensetzungsoberfläche mit Wasser oder wässrigen Lösungen, wie beispielsweise Körperflüssigkeiten, insbesondere Wundsekret, abbinden können. Dabei sollen die Zusammensetzungen Abbinden, ohne dass eine mechanische Vermischung mit Wasser oder wässrigen Lösungen notwendig ist. Die Zusammensetzung soll somit als Einkomponentensystem zur Herstellung eines Knochenersatzmaterials fungieren. Die Abbindung soll zu einem formstabilen Festkörper führen. Die Zusammensetzungen sollen aus toxikologisch möglichst unbedenklichen Materialien bestehen. Die Zusammensetzungen sollen möglichst nicht membranschädigend sein.

Es ist eine weitere Aufgabe der Erfindung, ein Verfahren zur Herstellung eines pharmazeutischen Wirkstoffträgers mittels einer derartigen Zusammensetzung bereitzustellen. Die pharmazeutischen Wirkstoffträger sollen als Knochenersatzmaterial zur lokalen Gabe eines pharmazeutischen Wirkstoffs verwendbar sein.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Zusammensetzung zur Herstellung eines Knochenersatzmaterials, welche mit Wasser oder einer wässrigen Lösung in einer zementartigen Abbindereaktion reagiert und einen Festkörper ausbildet, bestehend aus
a. mindestens einen in Wasser löslichen Polyether mit einer Schmelztemperatur von kleiner 25 °C, wobei der mindestens eine Polyether einen Wassergehalt von weniger als 1 Gewichts-% bezogen auf das Gesamtgewichts des mindestens einen Polyethers aufweist,
b. mindestens einem partikulären Calcium-haltigen Salz der Schwefelsäure und/oder der Phosphorsäure, welches bei Kontakt mit Wasser oder einer wässrigen Lösung in einer zementartigen Abbindereaktion reagiert und welches im Wesentlichen nicht in dem mindestens einem Polyether löslich ist,
c. mindestens einem partikulären Katalysator, der die Abbindereaktion der Zusammensetzung mit Wasser beschleunigt und welcher im Wesentlichen nicht in dem mindestens einem Polyether löslich ist,
d. 0 bis 10 Gewichts-% bezogen auf das Gesamtgewicht der Zusammensetzung mindestens eines pharmazeutischen Wirkstoffs,
e. 0 bis 30 Gewichts-% bezogen auf das Gesamtgewicht der Zusammensetzung mindestens eines partikulären, anorganischen Füllstoffs, welcher im Wesentlichen nicht in dem mindestens einem Polyether löslich ist.

In einer Ausführungsform ist die Zusammensetzung als eine plastisch verformbare Paste ausgebildet, welche bei Kontakt mit Wasser oder einer wässrigen Lösung an ihrer Oberfläche im Wesentlichen vollständig aushärtet. Diese Ausführungsform ist eine zweite Ausführungsform der Erfindung, welche vorzugsweise von der ersten Ausführungsform der Erfindung abhängt.

In einer Ausführungsform der Zusammensetzung weist der mindestens eine Polyether einen Anteil, insbesondere einen Gewichtsanteil von 20-40 Gewichts-%, vorzugsweise von 20-35 Gewichts-%, weiter bevorzugt von 20-30 Gewichts-%, am Gesamtgewicht der Zusammensetzung auf. Diese Ausführungsform ist eine dritte Ausführungsform der Zusammensetzung, welche vorzugsweise von der ersten oder der zweiten Ausführungsform der Erfindung abhängt.

In einer Ausführungsform der Zusammensetzung weist das mindestens eine Calcium-haltige Salz einen Anteil, insbesondere einen Gewichtsanteil, von 40-79 Gewichts-%, bevorzugt von 50-75 Gewichts-%, weiter bevorzugt von 55-65 Gewichts-%, am Gesamtgewicht der Zusammensetzung auf. Diese Ausführungsform ist eine vierte Ausführungsform der Erfindung, welche vorzugsweise von einer der vorhergehenden Ausführungsformen der Erfindung abhängt.

In einer Ausführungsform der Zusammensetzung ist das mindestens eine Calcium-haltige Salz Calciumsulfat-Hemidhydrat (CAS-Nr. 10034-76-1), Calciumsulfat-Anhydrit (CAS-Nr. 7778-18-9), Tricalciumphosphat (CAS-Nr. 7758-87-4), Tetracalciumphophat (CAS-Nr. 1306-01-0) oder eine Mischung der derselben, wobei Calciumsulfat-Hemihydrat (CAS-Nr. 10034-76-1) bevorzugt ist. Diese Ausführungsform ist eine fünfte Ausführungsform der Erfindung, welche vorzugsweise von einer der vorhergehenden Ausführungsformen der Erfindung abhängt.

In einer Ausführungsform der Zusammensetzung ist der mindestens eine Polyether ein Polyethylenglykol, bevorzugt Polyethylenglykol 200, Polyethylenglykol 400 und/oder Polyethylenglykol 600, weiter bevorzugt Polyethylenglykol 400. Diese Ausführungsform ist eine sechste Ausführungsform der Erfindung, welche vorzugsweise von einer der vorhergehenden Ausführungsformen der Erfindung abhängt.

In einer Ausführungsform der Zusammensetzung ist der mindestens eine Katalysator Calciumhydrogenphosphat, Ammoniumsulfat und/oder Kaliumsulfat, bevorzugt Ammoniumsulfat und/oder Kaliumsulfat, weiter bevorzugt Ammoniumsulfat oder Kaliumsulfat. Diese Ausführungsform ist eine siebte Ausführungsform der Erfindung, welche vorzugsweise von einer der vorhergehenden Ausführungsformen der Erfindung abhängt.

In einer Ausführungsform der Zusammensetzung ist der mindestens eine Füllstoff Magnesiumcarbonat, Hydroxylapatit und/oder Calciumcarbonat, bevorzugt Calciumcarbonat. Diese Ausführungsform ist eine achte Ausführungsform der Erfindung, welche vorzugsweise von einer der vorhergehenden Ausführungsformen der Erfindung abhängt.

In einer Ausführungsform der Zusammensetzung ist der mindestens eine pharmazeutische Wirkstoff ein antimikrobieller Wirkstoff, bevorzugt Gentamicin, Tobramycin, Amikacin, Vancomycin, Teicoplanin, Ramoplanin, Dalbavancin, Daptomycin, Clindamycin und/oder Fosfomycin, und/oder ein antimykotischer Wirkstoff, bevorzugt Amphotericin B, Fluconazol, Ketonazol, Miconazol und Griseofulvin, Caspofungin, Micafungin und/oder Anidulafungin. Diese Ausführungsform ist eine neunte Ausführungsform der Erfindung, welche vorzugsweise von einer der vorhergehenden Ausführungsformen der Erfindung abhängt.

In einer Ausführungsform der Zusammensetzung weist das Calcium-haltige Salz eine maximale Partikelgröße von 200 µm, bevorzugt von 100 µm, weiter bevorzugt von 64 µm auf. Diese Ausführungsform ist eine zehnte Ausführungsform der Erfindung, welche vorzugsweise von einer der vorhergehenden Ausführungsformen der Erfindung abhängt.

In einer Ausführungsform ist die Zusammensetzung im Wesentlichen frei, bevorzugt frei, von Tensiden. Diese Ausführungsform ist eine elfte Ausführungsform der Erfindung, welche vorzugsweise von einer der vorhergehenden Ausführungsformen der Erfindung abhängt.

Eine zwölfte Ausführungsform der Erfindung ist ein Verfahren zur Herstellung eines pharmazeutischen Wirkstoffträgers umfassend die Schritte
a. Bereitstellung einer Zusammensetzung nach einer der ersten bis elften Ausführungsformen der Erfindung,
b. Bereitstellen eines pharmazeutischen Wirkstoffs,
c. Mechanisches Vermischen der Zusammensetzung und des pharmazeutischen Wirkstoffs.

In einer Ausführungsform des Verfahrens ist der pharmazeutische Wirkstoff ein partikulärer pharmazeutischer Wirkstoff. Diese Ausführungsform ist eine dreizehnte Ausführungsform der Erfindung, welche vorzugsweise von der zwölften Ausführungsformen der Erfindung abhängt.

Eine vierzehnte Ausführungsform der Erfindung ist ein pharmazeutischer Wirkstoffträger hergestellt nach einer der zwölften oder dreizehnten Ausführungsform der Erfindung.

Eine fünfzehnte Ausführungsform der Erfindung ist eine Verwendung einer Zusammensetzung nach einer der ersten bis zwölften Ausführungsformen der Erfindung oder eines pharmazeutischen Wirkstoffträgers nach der vierzehnten Ausführungsform der Erfindung zur lokalen Freisetzung mindestens eines pharmazeutischen Wirkstoffs.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B. Eine "im Wesentlichen vollständig ausgehärtete Zusammensetzung" ist beispielsweise zu ≥95 bis ≤100 Gewichts-% bezogen auf Gesamtgewicht der Zusammensetzung ausgehärtet, wobei die noch nicht ausgehärteten Anteile entweder nicht ausgehärtet verbleiben oder im Laufe der Zeit nachträglich aushärten. Einer "im Wesentlichen von Substanz X freie Zusammensetzung" wird beispielsweise die Substanz X nicht aktiv zugesetzt. Unter einer "im Wesentlichen nicht in einer Flüssigkeit X löslichen Substanz Y" wird eine Substanz Y verstanden, welche sich bei Normalbedingungen (25 °C und 101,3 kPa) nicht mehr als bis zu 0,1 mol/l in der Flüssigkeit X löst.

### Ausführliche Beschreibung

Ein erster Gegenstand der Erfindung betrifft eine Zusammensetzung zur Herstellung eines Knochenersatzmaterials, welche mit Wasser oder einer wässrigen Lösung in einer zementartigen Abbindereaktion reagiert und einen Festkörper ausbildet, bestehend aus
a. mindestens einen in Wasser löslichen Polyether mit einer Schmelztemperatur von kleiner 25 °C, insbesondere mit einer Schmelztemperatur von kleiner 25 °C bei einem Druck von 101,3 kPa, wobei der mindestens eine Polyether einen Wassergehalt von weniger als 1 Gewichts-% bezogen auf das Gesamtgewichts des mindestens einen Polyethers aufweist,
b. mindestens einem partikulären Calcium-haltigen Salz der Schwefelsäure und/oder der Phosphorsäure, welches bei Kontakt mit Wasser oder einer wässrigen Lösung in einer zementartigen Abbindereaktion reagiert und welches im Wesentlichen nicht in dem mindestens einem Polyether löslich ist,
c. mindestens einem partikulären Katalysator, der die Abbindereaktion der Zusammensetzung mit Wasser beschleunigt und welcher im Wesentlichen nicht in dem mindestens einem Polyether löslich ist,
d. 0 bis 10 Gewichts-% bezogen auf das Gesamtgewicht der Zusammensetzung mindestens eines pharmazeutischen Wirkstoffs,
e. 0 bis 30 Gewichts-% bezogen auf das Gesamtgewicht der Zusammensetzung mindestens eines partikulären, anorganischen Füllstoffs, welcher im Wesentlichen nicht in dem mindestens einem Polyether löslich ist.

Die Erfindung betrifft eine Zusammensetzung. Unter einer Zusammensetzung wird erfindungsgemäß eine Mischung, vorzugsweise eine pastöse Mischung, bestehend aus den anspruchsgemäßen Bestandteilen a., b., c. und optional d. und/oder e. verstanden, welche mit Wasser oder einer wässrigen Lösung in einer zementartigen Abbindereaktion reagiert und einen Festkörper ausbildet. Die Zusammensetzung ist somit eine hydraulisch härtbare Zusammensetzung. Vorzugsweise übersteigt die Konsistenz der Zusammensetzung unter Normalbedingungen (25 °C und 101,3 kPa) nicht die einer knetbaren Masse, insbesondere einer händisch knetbaren Masse. Der aus der Zusammensetzung durch die zementartige Abbindereaktion erhaltbare Feststoff kann als Knochenersatzmaterial eingesetzt werden.

Die Bestandteile der Zusammensetzung addieren sich dabei zu 100 Gewichts-% bezogen auf das Gesamtgewicht der Zusammensetzung. Mit anderen Worten, die Zusammensetzung kann aus den Bestandteilen a., b. und c., aus den Bestandteilen a., b., c. und d., aus den Bestandteilen a., b., c. und e. oder aus den Bestandteilen a., b., c., d., und e. bestehen.

Die Zusammensetzung umfasst mit dem Bestandteil b. reaktive, partikuläre Calcium-haltige Salze der Schwefelsäure, der Phosphorsäure oder der Schwefelsäure und der Phosphorsäure, welche mit Wasser oder wässrigen Lösungen in einer zementartigen Abbindereaktion reagieren und somit einen Festkörper ausbilden. Während der zementartigen Abbindereaktion kommen die Bestandteile b. der Zusammensetzung mit Wasser oder einer wässrigen Lösung in Kontakt, so dass sich diese Bestandteile zumindest teilweise in Wasser auflösen und umkristallisieren bzw. in der wässrigen Phase wieder präzipitieren können und so einen, insbesondere zusammenhängenden, Festkörper ausbilden, welcher als Knochenersatzmaterial fungieren kann.

Als Kationen der Salze der Schwefelsäure und/oder der Phosphorsäure kommt bzw. kommen erfindungsgemäß ein oder mehrere Calcium-Ionen zum Einsatz. Calcium hat dabei den Vorteil, dass es, im Gegensatz zu anderen Kationen, wie beispielsweise Magnesium, physiologisch unbedenklicher ist. Dabei wird unter einem Calcium-haltigen Salz ein Salz verstanden, welches hauptsächlich Calcium als Kation bzw. Kationen enthält. Eingeschlossen sind dabei auch solche Salze, welche weitere Kationen, insbesondere Na, K, NH₄ oder Mg, eventuell auch als Verunreinigungen enthalten, wobei reine Calcium-haltige Salze bevorzugt sind.

Die Zusammensatzung enthält als Bestandteil c. mindestens einen partikulären Katalysator, der die Abbindereaktion der Zusammensetzung mit Wasser beschleunigt. Der mindestens eine Katalysator wird in Abhängigkeit des Bestandteils b. der Zusammensetzung gewählt, da unterschiedliche Ca-haltige Salze der Schwefelsäure und/oder der Phosphorsäure sich unterschiedlich schnell mit bestimmten Katalysatoren in der zementartigen Abbindereaktion abbinden.

Als einen optionalen Bestandteil enthält die Zusammensetzung mit Bestandteilt d. einen pharmazeutischen Wirkstoff, welcher nach dem Abbinden aus der abgebundenen Zusammensetzung an der applizierten Stelle im Patienten abgegeben werden kann. Als geeignete pharmazeutische Wirkstoffe kommen alle für die lokale Behandlung von Knochenerkrankungen und den angrenzenden Geweben relevanten Substanzen in Betracht. Die pharmazeutischen Wirkstoffe können die Funktion des Knochenersatzmaterials in gewünschter Weise unterstützen. Dabei können partikuläre, wie beispielsweise flockenartige und/oder lyophilisierte, und/oder flüssige pharmazeutische Wirkstoffe zum Einsatz kommen.

Als einen weiteren optionalen Bestandteil enthält die Zusammensetzung mit Bestandteil e. mindestens einen partikulären, anorganischen Füllstoff. Ein Füllstoff wird insbesondere in die sich beim Abbinden formende Zementmatrix eingebunden, ist aber im Wesentlichen nur mittelbar an der zementartigen Abbindereaktion beteiligt. Die Teilnahme kann beispielsweise darin bestehen, dass ein Füllstoff als ein Kristallisationskeim für die Mineralisation während der zementartigen Abbindereaktion fungiert und damit insbesondere die Abbindekinetik beeinflusst, aber selbst im Wesentlichen nicht umgesetzt wird.

Um die Bestandteile b., c. und optional d. und/oder e. in eine formbare, vorzugsweise bei Normalbedingungen knetbare, Masse zusammenzuführen, enthält die Zusammensetzung einen in Wasser löslichen Polyether mit einer Schmelztemperatur von kleiner 25 °C, also bei Normalbedingungen flüssigen Polyether, wobei der mindestens eine Polyether einen Wassergehalt von weniger als 1 Gewichts-% bezogen auf das Gesamtgewichts des mindestens einen Polyethers aufweist. Der mindestens eine Polyether benetzt die partikulären Bestandteile der Zusammensetzung zumindest teilweise, vorzugsweise im Wesentlichen vollständig, so dass eine formbare, vorzugsweise homogene, Masse entsteht, welche in eine Kavität, insbesondere in eine Kavität eines Knochens, einbringbar ist und dort aufgrund ihrer Viskosität verbleibt, bis sie mit Wasser oder einer wässrigen Lösung, insbesondere einer Körperflüssigkeit, wie beispielsweise Wundsekret, in der Kavität in einer zementartigen Abbindereaktion zu einem Festköper abbindet und so als Knochenersatzmaterial fungiert.

Unter einem Polyether werden Substanzen verstanden, welche mindestens vier Ethergruppen im einzelnen Molekül umfassen. Vorzugsweise sind die Polyether lineare Moleküle mit mindestens vier Ethergruppen.

Es ist wesentlich, dass der Polyether einen Wassergehalt von weniger als 1 Gewichts-%, vorzugsweise weniger als 0,5 Gewichts-%, bezogen auf das Gesamtgewichts des mindestens einen Polyethers aufweist, damit die Zusammensetzung lagerstabil ist, bis sie an ihrem gewünschten Applikationsort zum Abbinden appliziert wird. Vorzugsweise ist der Polyether im Wesentlichen frei von Wasser.

Neben etwaigen optionalen flüssigen pharmazeutischen Wirkstoffen und/oder etwaigen Verunreinigungen enthält die Zusammensetzung somit keine (weiteren) Flüssigkeiten, insbesondere keine nicht wasserlöslichen Flüssigkeiten, außer dem mindestens einen Polyether. Die erfindungsgemäßen Polyether sind, bevorzugt in beliebigen Mischungsverhältnissen, in Wasser löslich. Dies erlaubt ein schnelles Eindringen von Wasser oder wässrigen Lösungen in die Zusammensetzung bzw. einen, zumindest teilweisen, vorzugsweise im Wesentlichen vollständigen, Austausch des Polyethers der Zusammensetzung gegen Wasser oder eine wässrige Lösung nach einer Applikation der Zusammensetzung in einen Patienten, wodurch es zu der zementartigen Abbindereaktion unter Ausbildung eines Festkörpers kommt. Dieser Festkörper dient als Knochenersatzmaterial. Der mindestens eine Polyether erlaubt dabei ein im Wesentlichen vollständiges Abbinden der Zusammensetzung, auch wenn diese lediglich an ihrer Oberfläche, oder zumindest einem Teil ihrer Oberfläche, mit Wasser oder einer wässrigen Lösung, also mit Körperflüssigkeiten eines Patienten, in Kontakt gebracht wird. Ein, insbesondere mechanisches, Vermischen der Zusammensetzung mit Wasser oder einer wässrigen Lösung ist nicht notwendig, um die Zusammensetzung im Wesentlichen vollständig abzubinden. Weiterhing ist die Zusammensetzung auch bei lediglich oberflächlichem Kontakt mit Wasser oder einer wässrigen Lösung durch ihre Bestandteile, insbesondere durch die ausschließliche Verwendung des mindestens einen Polyethers, innerhalb weniger Minuten, beispielsweise innerhalb von 5 Minuten, zumindest an ihrer Oberfläche, insbesondere bis zu einer Schichtdicke von mehr als 1 mm, in einen Festkörper abbindbar. Die genaue Zeit bis zum im Wesentlichen vollständigen Ausbilden eines Festkörpers hängt erwartungsgemäß von der Menge der abzubindenden Zusammensetzung, deren gegebener Form, insbesondere ihrem Verhältnis von Oberfläche zu Massen, und ihrer Exposition zu Wasser oder einer wässrigen Lösung ab.

Überraschenderweise sind die Zusammensetzungen, trotz der, insbesondere ausschließlichen, Verwendung des mindestens eines Polyethers als wasserlöslicher Flüssigkeit, über einen längeren Zeitraum, beispielsweise über mehrere, Monate, wie beispielsweise mehr als drei Monate, lagerstabil, bis sie bei Inkontaktbringen mit Wasser oder einer wässrigen Lösung in einer zementartigen Abbindereaktion reagieren. Lagerstabil sind die Zusammensetzungen beispielsweise deshalb, falls die ursprüngliche Viskosität der Zusammensetzungen im Wesentlichen unverändert und/oder die Zusammensetzungen über die Lagerdauer als geschmeidig knet- und formbare Masse verbleiben. Andere wasserlösliche Flüssigkeiten als Trägermaterial in derartigen Zusammensetzungen, wie beispielsweise Glycerin, erwiesen sich hingegen nicht als so lange lagerstabil.

Um die Zusammensetzung als formbare Masse zur Verfügung zu stellen, welche aufgrund ihrer Viskosität anwendungssicher in Kavitäten eines Patienten eingebracht werden kann und welche mit Wasser oder mit einer wässrigen Flüssigkeit unter einer zementartigen Abbindereaktion reagiert, sind die partikulären Bestandteile der Zusammensetzung, insbesondere die Bestandteile b., c. und, falls vorhanden, e. im Wesentlichen nicht in dem mindestens einem Polyether löslich.

Bestimmt durch die Gewichtsanteile der Zusammensetzung, insbesondere dem Verhältnis von partikulären zu flüssigen Bestandteilen der Zusammensetzung, kann diese vor der zementartigen Abbindereaktion in unterschiedlichen Viskositäten vorliegen.

Eine Ausführungsform der Zusammensetzung ist dadurch gekennzeichnet, dass die Zusammensetzung als eine plastisch verformbare Paste ausgebildet ist, welche bei Kontakt mit Wasser oder einer wässrigen Lösung an ihrer Oberfläche im Wesentlichen vollständig aushärtet. Eine plastisch verformbare Paste weist, im Gegenzug zu einer niederviskoseren Masse den Vorteil auf, dass diese in eine Kavität derart einbringbar ist, ohne dass die Zusammensetzung aus dieser Kavität allein aufgrund der Schwerkraft wieder ausbringbar ist. Eine plastisch verformbare Paste weist weiterhin eine Viskosität auf, welche ein händisches Verformen zulässt. Somit ist die Zusammensetzung auch ohne Werkzeuge, wie beispielsweise ein Messer zum Schneiden oder Schnitzen, in beliebige Formen überführbar. Dies erleichtert einem Operateur die Verwendung der Zusammensetzung im Zuge einer Operation.

Eine Ausführungsform der Zusammensetzung ist dadurch gekennzeichnet, dass der mindestens eine Polyether einen Anteil, insbesondere einen Gewichtsanteil, von 20-40 Gewichts-%, vorzugsweise von 20-30 Gewichts-%, am Gesamtgewicht der Zusammensetzung aufweist. Dies erlaubt ein im Wesentlichen homogenes Anmischen der Bestandteile der Zusammensetzung, so dass im Wesentlichen alle partikulären Bestandteile zumindest teilweise, vorzugsweise im Wesentlichen vollständig, von dem mindestens einen Polyether ummantelt sind, was ein möglichst vollständiges zementartiges Abbinden bei Kontakt der Zusammensetzung auch bei lediglich oberflächlichem Kontakt mit Wasser oder einer wässrigen Lösung ermöglicht. Weiterhin weist dadurch die Zusammensetzung eine möglichst formbare, vorzugsweise pastöse Viskosität auf, welche eine Applikation der Zusammensetzung in einen Patienten erleichtert.

Eine Ausführungsform der Zusammensetzung ist dadurch gekennzeichnet, dass das mindestens eine Calcium-haltige Salz einen Anteil, insbesondere einen Gewichtsanteil, von 40-79 Gewichts-% am Gesamtgewicht der Zusammensetzung aufweist. Derartige Zusammensetzungen ergeben nach der zementartigen Abbindereaktion einen stabilen Festkörper, welcher gut als Knochenersatzmaterial fungieren kann.

Als das mindestens eine Calcium-haltige Salz der Schwefelsäure und/oder der Phosphorsäure können unterschiedliche Salze oder deren Mischungen verwendet werden, solange diese eine zementartige Abbindereaktion mit Wasser oder einer wässrigen Lösung unter Ausbildung eines Festkörpers eingehen können.

Eine Ausführungsform der Zusammensetzung ist dadurch gekennzeichnet, dass das mindestens eine Calcium-haltige Salz Calciumsulfat-Hemihydrat, Calciumsulfat-Anhydrit, Tricalciumphosphat, Tetracalciumphosphat oder beliebige Mischungen der vorgenannten Salze ist, wobei Calciumsulfat-Hemihydrat bevorzugt ist.

Als der mindestens eine Polyether können beliebige wasserlösliche, bevorzugt in beliebigen Mischungsverhältnissen mit Wasser lösliche, Polyether in Reinform oder in beliebigen Mischungen zum Einsatz kommen, solange diese eine Schmelztemperatur von kleiner 25 °C, und insbesondere eine Schmelztemperatur von kleiner 25 °C bei 101,3 kPa, aufweisen. Beispielsweise können Polymere des Propylenglycols, also Polypropylenglycole, verwendet werden. Beispielsweise können Polypropylenglycol 200 bis Polypropylenglycol 600.

Eine Ausführungsform der Zusammensetzung ist dadurch gekennzeichnet, dass der mindestens eine Polyether ein Polyethylenglykol, eine Mischung verschiedener Polyethylenglykole oder eine Mischung eines Polyethylenglykol mit einem Polypropylenglykol ist, wobei ein reines Polyethylenglykol bevorzugt ist. Polyethylenglykole sind bevorzugt, da diese günstig, leicht zugänglich und insbesondere biologisch gut verträgliche wasserlösliche Polyether darstellen. Besonders bevorzugte Polyethylenglykole sind Polyethylenglykol 200, Polyethylenglykol 400 und Polyethylenglykol 600, wobei von diesen Polyethylenglykol 400 am bevorzugtesten ist. Polyethylenglykol 400 ist bevorzugt, da dieses aufgrund seiner Viskosität eine gut handhabbare Zusammensetzung ergibt.

Als Katalysator können unterschiedliche Substanzen in Reinform oder in Mischungen zugesetzt werden, um die zementartige Abbindereaktion der Zusammensetzung zu beschleunigen.

Eine Ausführungsform der Zusammensetzung ist dadurch gekennzeichnet, dass der mindestens eine Katalysator Calciumdihydrogenphosphat (CAS-7758-23-8), Ammoniumsulfat (CAS-7783-20-2), Kaliumsulfat (CAS-Nr. 7778-80-5) oder eine Mischung aus zwei oder mehr der vorgenannten Substanzen ist. Der mindestens eine Katalysator wird vorzugsweise in Abhängigkeit des oder der verwendeten Ca-haltigen Salze ausgewählt.

Der mindestens eine Katalysator kann der Zusammensetzung in unterschiedlichen Gewichtsanteilen zugesetzt werden, um die zementartige Abbindereaktion zu beschleunigen.

Eine Ausführungsform der Zusammensetzung ist dadurch gekennzeichnet, dass der Katalysator einen Anteil, insbesondere einen Gewichtsanteil, von 1-12 Gewichts-% am Gesamtgewicht der Zusammensetzung aufweist. Geringere Anteile als 1 Gewichts-% an Katalysator verlangsamen die zementartige Abbindereaktion, so dass sich unter Umständen eine Operation unnötig in die Länge zieht und ein Risiko besteht, dass sich die in den Patienten applizierte Zusammensetzung vor der Ausbildung des Festkörpers von der gewünschten Stelle entfernt oder eine ungewünschte Form annimmt. Höhere Anteile an Katalysator beschleunigen zwar die Ausbildung des Festkörpers, allerdings trägt der Katalysator nur bedingt zur Festigkeit des Festkörpers bei, so dass dieser bei höheren Anteilen des Katalysators als 12 Gewichts-% an Festigkeit einbüßen kann. Zudem ist eine noch stärker beschleunigte zementartige Abbindereaktion üblicherweise nicht praxisrelevant.

Als partikuläre, anorganische Füllstoffe können unterschiedliche Substanzen in Reinform oder in Mischungen der Zusammensetzung zugesetzt werden.

Eine Ausführungsform der Zusammensetzung ist dadurch gekennzeichnet, dass der mindestens eine Füllstoff Magnesiumcarbonat, Hydroxylapatit, Calciumcarbonat oder eine beliebige Mischung aus zwei oder mehr der vorgenannten Substanzen ist, wobei Calciumcarbonat bevorzugt ist. Calciumcarbonat ist bevorzugt, da dieses besonders bioverträglich ist.

Als der optionale mindestens eine pharmazeutische Wirkstoff können unterschiedliche Substanzen in Reinform oder in Mischungen der Zusammensetzung zugesetzt werden, welche für eine lokale Behandlung von Knochenerkrankungen und/oder den angrenzenden Geweben in Betracht kommen.

Eine Ausführungsform der Zusammensetzung ist dadurch gekennzeichnet, dass der mindestens eine pharmazeutische Wirkstoff ein antimikrobieller Wirkstoff, ein antimykotischer Wirkstoff oder eine Mischung aus mindestens einem antimikrobiellem und mindestens einem antimykotischem Wirkstoff ist. Dabei sind als antimikrobielle Wirkstoffe Gentamicin, Tobramycin, Amikacin, Vancomycin, Teicoplanin, Ramoplanin, Dalbavancin, Daptomycin, Clindamycin und Fosfomycin sowie als antimykotische Wirkstoffe Amphotericin B, Fluconazol, Ketonazol, Miconazol und Griseofulvin, Caspofungin, Micafungin und Anidulafungin bevorzugt.

Das mindestens eine Calcium-haltige Salz der Schwefelsäure und/oder der Phosphorsäure kann unterschiedliche Partikelgrößen aufweisen.

Eine Ausführungsform der Zusammensetzung ist dadurch gekennzeichnet, dass das mindestens eine Calcium-haltige Salz eine maximale Partikelgröße von 200 µm, bevorzugt von maximal 100 µm, weiter bevorzugt von maximal 64 µm, aufweist. Die maximale Partikelgröße kann dabei beispielsweise durch die Wahl entsprechender Siebfraktionen der jeweiligen Calcium-haltigen Salze erhalten werden.

Derartige Partikelgrößen sorgen für eine möglichst homogene Mischung der Zusammensetzung und ermöglichen eine möglichst schnelle und gleichmäßige und Ausbildung eines Feststoffs durch die zementartige Abbindereaktion der Zusammensetzung mit Wasser oder einer wässrigen Lösung.

Eine Ausführungsform der Zusammensetzung ist dadurch gekennzeichnet, dass die Zusammensetzung im Wesentlichen frei von Tensiden ist. Derartige Zusammensetzungen bestehen nur aus solchen Bestandteilen a., b., c. und optional d. und/oder e., welche im Wesentlichen keine tensidischen Eigenschaften aufweisen. Zusammensetzung ohne Tenside sind bevorzugt, da Tenside eine membranschädigende Wirkung besitzen und so negative Auswirkungen auf die mit der Zusammensetzung zu behandelnden Patienten zeigen können. Der Begriff "Tensid" umfasst insbesondere amphiphile Moleküle, welche auch als Emulgatoren oder Netzmittel wirken können.

Ein weiterer Gegenstand der Erfindung betrifft ein Verfahren zur Herstellung eines pharmazeutischen Wirkstoffträgers umfassend die Schritte
a. Bereitstellung einer Zusammensetzung nach einer der vorhergehenden Ausführungsformen,
b. Bereitstellen eines pharmazeutischen Wirkstoffs,
c. Mechanisches Vermischen der Zusammensetzung und des pharmazeutischen Wirkstoffs.

Die in Schritt a. des Verfahrens bereitgestellte Zusammensetzung kann dabei bereits als Bestandteil d. einen oder mehrere pharmazeutische Wirkstoffe enthalten oder frei von pharmazeutischen Wirkstoffen sein.

Der in Schritt b. des Verfahrens bereitgestellte pharmazeutische Wirkstoff ist bevorzugt einer oder mehrere der bereits für die Zusammensetzung offenbarten pharmazeutischen Wirkstoffe. Falls die in Schritt a. bereitgestellte Zusammensetzung bereits als Bestandteil d. einen pharmazeutischen Wirkstoff oder eine Mischung von pharmazeutischen Wirkstoffen enthält, kann der in Schritt b. des Verfahrens bereitgestellte pharmazeutische Wirkstoff auch der gleiche Wirkstoff oder die gleiche Mischung von pharmazeutischen Wirkstoffen sein. In diesem Fall dient das Verfahren einer Erhöhung der Konzentration des entsprechenden pharmazeutischen Wirkstoffs oder der entsprechenden Mischung von pharmazeutischen Wirkstoffen in der bereitgestellten Zusammensetzung.

In Schritt c. des Verfahrens erfolgt ein mechanisches Vermischen der in Schritt a. bereitgestellten Zusammensetzung und des in Schritt b. bereitgestellten pharmazeutischen Wirkstoffs unter Bereitstellung eines pharmazeutischen Wirkstoffträgers.

Das mechanische Vermischen kann auf unterschiedliche Weise bewerkstelligt werden. Beispielsweise kann dies unter Verwendung eines Hilfsmittels, wie beispielsweise eines Spatels oder eines anderen geeigneten Mischwerkzeugs, durchgeführt werden. Vorzugsweise erfolgt das mechanische Vermischen händisch und daher ohne Hilfsmittel, da dies für einen Operateur schnell und einfach auch während einer Operation durchgeführt werden kann. Ein händisches Mischen kann auch als Verkneten bezeichnet werden.

Der in Schritt b. des Verfahrens bereitgestellte pharmazeutische Wirkstoff kann flüssig sein.

Eine Ausführungsform des Verfahrens ist dadurch gekennzeichnet, dass der pharmazeutische Wirkstoff ein partikulärer Wirkstoff ist. Beispielsweise kann der pharmazeutische Wirkstoff pulverförmig oder flockenförmige sein. In einer Ausführungsform ist der pharmazeutische Wirkstoff lyophilisiert. Partikuläre pharmazeutische Wirkstoffe sind für einen Operateur in einer laufenden Operation leichter zu handhaben als flüssige pharmazeutische Wirkstoffe, insbesondere wenn das mechanische Vermischen in Schritt c. des Verfahrens händisch durchgeführt wird.

Ein weiterer Gegenstand der Erfindung betrifft einen pharmazeutischen Wirkstoffträger hergestellt nach einem derartigen Verfahren.

Eine Zusammensetzung enthaltend einen pharmazeutischen Wirkstoff und ein mit dem Verfahren hergestellter pharmazeutischer Wirkstoffträger können sich, außer offensichtlichen Unterschieden in ihren Bestandteilen, unter Umständen dadurch unterscheiden, dass der durch das Verfahren zur Zusammensetzung beigemischte pharmazeutische Wirkstoff weniger homogen in der Zusammensetzung verteilt ist, als wäre dieser bereits vor dem Verfahren in der Zusammensetzung enthalten. Würde der pharmazeutische Wirkstoff bereits bei der Herstellung der Zusammensetzung zugefügt, könnte dieser mit den partikulären Bestandteilen der Zusammensetzung vorvermischt werden, bevor durch Zugabe des mindestens einen Polyethers die Zusammensetzung in eine formbare Masse überführt wird. Ein "nachträgliches" Vermischen des pharmazeutischen Wirkstoffs in diese formbare Masse kann, je nach Viskosität der Zusammensetzung, erschwert sein. Ein Vorteil des pharmazeutischen Wirkstoffträgers ist, dass dieser gezielt auf die Bedürfnisse eines Patienten direkt im Operationssaal abgestimmt und bereitgestellt werden kann.

Ein weiterer Gegenstand der Erfindung betrifft eine Verwendung einer Zusammensetzung nach einer der vorgenannten Ausführungsformen oder eines pharmazeutischen Wirkstoffträger nach einer der vorgenannten Ausführungsformen zu lokalen Freisetzung mindestens eines pharmazeutischen Wirkstoffs, insbesondere zur Behandlung von Knochenerkrankungen und den an Knochen angrenzenden Geweben.

Ein Vorteil der Verwendung des pharmazeutischen Wirkstoffträgers ist seine flexible Besetzung mit pharmazeutischen Wirkstoffen, auch und gerade, wenn dieser in einer laufenden Operation hergestellt wird. Ein Operateur ist somit in der Lage, schnell und einfach einen, eventuell zusätzlichen, pharmazeutischen Wirkstoff der Zusammensetzung zuzufügen. Eventuelle Inhomogenitäten fallen gegenüber diesem Vorteil weniger ins Gewicht.

Die für die Zusammensetzung offenbarten Merkmale sind auch für das Verfahren und den pharmazeutischer Wirkstoffträger offenbart und umgekehrt.

### Beispiele

Die Erfindung wird im Folgenden durch Beispiele weiter beispielhaft illustriert. Die Erfindung ist nicht auf die Beispiele beschränkt.

Es wurden folgende Materialien für die Herstellung der Beispiele der Zusammensetzungen zur Herstellung eines Knochenersatzmaterials verwendet:
CSH: Calciumsulfat-Hemihydrat (CAS-Nr. 10034-76-1)
CSAH: Calciumsulfat (Anhydrit) (CAS-Nr. 7778-18-9)
AS: Ammoniumsulfat (CAS-7783-20-2)
KS: Kaliumsulfat (CAS-Nr. 7778-80-5)
CA: Calciumcarbonat (CAS-Nr. 471-34-1)
HA: Hydroxylapatit (CAS-Nr. 1306-06-5)
GS: Gentamicinsulfat
VHCI: Vancomycinhydrochlorid
CL: Clindamycinhydrochlorid
TCP: Tricalciumphosphat (CAS-Nr. 7758-87-4)
CDHP: Calciumdihydrogenphosphat (CAS-7758-23-8)
TTCP: Tetracalciumphosphat (CAS-Nr. 1306-01-0)

Die Knochenersatzmaterialien wurden hergestellt, indem zuerst die partikulären Komponenten der entsprechenden Zusammensetzungen mittels Porzellankugeln als Mahlkörper mit Hilfe eines Turbula-Mischers vermahlen wurden. Im Anschluss wurde das so erhaltene partikuläre Gemisch mit den jeweiligen Flüssigkeiten (Polyethylenglykol 200 (PEG200) oder Polyethylenglykol 400 (PEG400) für die erfindungsgemäßen Ausführungsbeispiele 1 bis 46 beziehungsweise Glycerinacetat, Oleinsäure oder Miglyol^{®} 812 für die Vergleichsbeispiele 1 bis 10), unter Erhalt der Zusammensetzungen in Form viskoser Pasten vermischt. Aus den so erhaltenen Zusammensetzungen wurden dann händisch Kugeln mit einem Durchmesser von ca. 10 mm geformt, welche in destilliertem Wasser eingelagert wurden. Das Fortschreiten der zementartigen Abbindereaktion der Zusammensetzungen im Wasser wurde durch Überprüfen der Festigkeit der Kugeln überwacht. Dazu wurde die Festigkeit der Kugeln minütlich durch Einstechen mit einem Spatel geprüft.

Die Zusammensetzungen der Ausführungsbeispiele ohne Zugabe eines pharmazeutischen Wirkstoffs waren im Wesentlichen ohne Änderung der ursprünglichen Viskositäten und ohne auszuhärten für mindestens 3 Monat lagerbar.

Die Kugeln der Ausführungsbeispiele 1 bis 46 waren alle nach ca. 5 Minuten zumindest an deren Oberfläche ausgehärtet. Die zementartige Abbindereaktion im Inneren der Kugeln erfolgte dann innerhalb weiterer weniger Minuten. Weiterhin behielten die aus den Zusammensetzungen geformten Kugeln beim Abhärten im Wesentlichen ihre Form und Größe. Die Zusammensetzungen härten also im Wesentlichen ohne signifikante Volumenänderung aus.

Bei den Kugeln der Vergleichsbeispiele kam es im Zuge der Aushärtung zu einer Volumenzunahme in einem Bereich von ca. 5 bis 15 Volumenprozent bezogen auf das ursprüngliche Volumen der Kugeln. Weiterhin kam es zu einem Einreißen der Oberflächen der Kugeln.

| Ausführungsbeispiel | CSH [g] | AS [g] | PEG200 | GS [g] | VHCL [g\|] | CL [g] |
|---|---|---|---|---|---|---|
| 1 | 8,5 | 1,5 | 3,0 | - | - | - |
| 2 | 9,0 | 1,0 | 3,0 | - | - | - |
| 3 | 9,5 | 0,5 | 3,0 | - | - | - |
| 4 | 9,5 | 0,5 | 3,0 | 0,5 | - | - |
| 5 | 9,5 | 0,5 | 3,0 | 0,3 | - | - |
| 6 | 9,5 | 0,5 | 3,0 | 0,1 | - | - |
| 7 | 9,5 | 0,5 | 3,0 | - | 0,5 | - |
| 8 | 9,5 | 0,5 | 3,0 | - | 0,3 | - |
| 9 | 9,5 | 0,5 | 3,0 | - | 0,1 | - |
| 10 | 9,5 | 0,5 | 3,0 | - | - | 0,5 |
| 11 | 9,5 | 0,5 | 3,0 | - | - | 0,3 |
| 12 | 9,5 | 0,5 | 3,0 | - | - | 0,1 |

| Ausführungsbeispiel | CSH [g] | AS [g] | PEG400 | GS [g] | VHCL [g\|] | CL [g] |
|---|---|---|---|---|---|---|
| 13 | 8,5 | 1,5 | 3,0 | - | - | - |
| 14 | 9,0 | 1,0 | 3,0 | - | - | - |
| 15 | 9,5 | 0,5 | 3,0 | - | - | - |
| 16 | 9,5 | 0,5 | 3,0 | 0,5 | - | - |
| 17 | 9,5 | 0,5 | 3,0 | 0,3 | - | - |
| 18 | 9,5 | 0,5 | 3,0 | 0,1 | - | - |
| 19 | 9,5 | 0,5 | 3,0 | - | 0,5 | - |
| 20 | 9,5 | 0,5 | 3,0 | - | 0,3 | - |
| 21 | 9,5 | 0,5 | 3,0 | - | 0,1 | - |
| 22 | 9,5 | 0,5 | 3,0 | - | - | 0,5 |
| 23 | 9,5 | 0,5 | 3,0 | - | - | 0,3 |
| 24 | 9,5 | 0,5 | 3,0 | - | - | 0,1 |

| Ausführungsbeispiel | CSAH [g] | AS [g] | PEG200 | CA [g] | TCP [g\|] | HA [g] | GS [g] |
|---|---|---|---|---|---|---|---|
| 25 | 6,0 | 0,5 | 3,0 | 3,5 | - | - | 0,5 |
| 26 | 6,0 | 0,5 | 3,0 | - | 3,5 | - | 0,5 |
| 27 | 6,0 | 0,5 | 3,0 | - | - | 3,5 | 0,5 |

| Ausführungsbeispiel | CSH [g] | KS [g] | PEG200 | GS [g] | VHCL [g\|] | CL [g] |
|---|---|---|---|---|---|---|
| 28 | 8,5 | 1,5 | 3,0 | - | - | - |
| 29 | 9,0 | 1,0 | 3,0 | - | - | - |
| 30 | 9,5 | 0,5 | 3,0 | - | - | - |
| 31 | 9,5 | 0,5 | 3,0 | 0,5 | - | - |
| 32 | 9,5 | 0,5 | 3,0 | 0,3 | - | - |
| 33 | 9,5 | 0,5 | 3,0 | 0,1 | - | - |
| 34 | 9,5 | 0,5 | 3,0 | - | 0,5 | - |
| 35 | 9,5 | 0,5 | 3,0 | - | 0,3 | - |
| 36 | 9,5 | 0,5 | 3,0 | - | 0,1 | - |
| 37 | 9,5 | 0,5 | 3,0 | - | - | 0,5 |
| 38 | 9,5 | 0,5 | 3,0 | - | - | 0,3 |
| 39 | 9,5 | 0,5 | 3,0 | - | - | 0,1 |

| Ausführungsbeispiel | TCP [g] | TTCP [g] | CDHP [g] | PEG200 [g] | GS [g] | VHCI [g] |
|---|---|---|---|---|---|---|
| 40 | 6,00 | - | 4,00 | 4,00 | - | - |
| 41 | 6,00 | - | 4,00 | 4,00 | - | - |
| 42 | 6,00 | - | 4,00 | 4,00 | 0,74 | - |
| 43 | 6,00 | - | 4,00 | 4,00 | - | 0,74 |
| 44 | - | 5,00 | 5,00 | 4,00 | - | - |
| 45 | - | 5,50 | 4,50 | 4,00 | - | - |
| 46 | - | 5,50 | 4,50 | 4,00 | 0,74 | - |

| Vergleichsbeispiel | CSH [g] | AS [g] | Miglyol^{®} 812 [g] | GS [g] |
|---|---|---|---|---|
| 1 | 9,5 | 0,5 | 3,0 | 0,5 |
| 2 | 9,5 | 0,5 | 3,0 | 0,3 |
| 3 | 9,5 | 0,5 | 3,0 | 0,1 |

| Vergleichsbeispiel | CSH [g] | AS [g] | Glycerintriacetat [g] | GS [g] |
|---|---|---|---|---|
| 4 | 9,5 | 0,5 | 3,0 | 0,5 |
| 5 | 9,5 | 0,5 | 3,0 | 0,3 |
| 6 | 9,5 | 0,5 | 3,0 | 0,1 |

| Vergleichsbeispiel | CSH [g] | AS [g] | Oleinsäure [g] | GS [g] |
|---|---|---|---|---|
| 7 | 9,5 | 0,5 | 3,0 | 0,5 |
| 8 | 9,5 | 0,5 | 3,0 | 0,3 |
| 9 | 9,5 | 0,5 | 3,0 | 0,1 |

| Vergleichsbeispiel | TCP [g] | CDHP [g] | Miglyol^{®} 812 [g] | GS [g] |
|---|---|---|---|---|
| 10 | 6,00 | 4,0 | 4,00 | 0,74 |

## Patentansprüche

1. Zusammensetzung zur Herstellung eines Knochenersatzmaterials, welche mit Wasser oder einer wässrigen Lösung in einer zementartigen Abbindereaktion reagiert und einen Festkörper ausbildet, bestehend aus
a. mindestens einen in Wasser löslichen Polyether mit einer Schmelztemperatur von kleiner 25 °C, wobei der mindestens eine Polyether einen Wassergehalt von weniger als 1 Gewichts-% bezogen auf das Gesamtgewichts des mindestens einen Polyethers aufweist,
b. mindestens einem partikulären Calcium-haltigen Salz der Schwefelsäure und/oder der Phosphorsäure, welches bei Kontakt mit Wasser oder einer wässrigen Lösung in einer zementartigen Abbindereaktion reagiert und welches im Wesentlichen nicht in dem mindestens einem Polyether löslich ist,
c. mindestens einem partikulären Katalysator, der die Abbindereaktion der Zusammensetzung mit Wasser beschleunigt und welcher im Wesentlichen nicht in dem mindestens einem Polyether löslich ist,
d. 0 bis 10 Gewichts-% bezogen auf das Gesamtgewicht der Zusammensetzung mindestens eines pharmazeutischen Wirkstoffs,
e. 0 bis 30 Gewichts-% bezogen auf das Gesamtgewicht der Zusammensetzung mindestens eines partikulären, anorganischen Füllstoffs, welcher im Wesentlichen nicht in dem mindestens einem Polyether löslich ist.

2. Zusammensetzung nach Anspruch 1, wobei Zusammensetzung als eine plastisch verformbare Paste ausgebildet ist, welche bei Kontakt mit Wasser oder einer wässrigen Lösung an ihrer Oberfläche im Wesentlichen vollständig aushärtet.

3. Zusammensetzung nach Anspruch 1 oder 2, wobei der mindestens eine Polyether einen Anteil von 20-40 Gewichts-% am Gesamtgewicht der Zusammensetzung aufweist.

4. Zusammensetzung nach einem der vorhergehenden Ansprüche, wobei das mindestens eine Calcium-haltige Salz einen Anteil von 40-79 Gewichts-% am Gesamtgewicht der Zusammensetzung aufweist.

5. Zusammensetzung nach einem der vorhergehenden Ansprüche, wobei das mindestens eine Calcium-haltige Salz Calciumsulfat-Hemihydrat, Calciumsulfat-Anhydrit Tricalciumphosphat und/oder Tetracalciumphosphat ist.

6. Zusammensetzung nach einem der vorhergehenden Ansprüche, wobei der mindestens eine Polyether ein Polyethylenglykol, bevorzugt Polyethylenglykol 200, Polyethylenglykol 400 und/oder Polyethylenglykol 600, ist.

7. Zusammensetzung nach einem der vorhergehenden Ansprüche, wobei der mindestens eine Katalysator Calciumdihydrogenphosphat, Ammoniumsulfat und/oder Kaliumsulfat ist.

8. Zusammensetzung nach einem der vorhergehenden Ansprüche, wobei der mindestens eine Füllstoff Magnesiumcarbonat, Hydroxylapatit und/oder Calciumcarbonat ist.

9. Zusammensetzung nach einem der vorhergehenden Ansprüche, wobei der mindestens eine pharmazeutische Wirkstoff ein antimikrobieller Wirkstoff, bevorzugt Gentamicin, Tobramycin, Amikacin, Vancomycin, Teicoplanin, Ramoplanin, Dalbavancin, Daptomycin, Clindamycin und/oder Fosfomycin, und/oder ein antimykotischer Wirkstoff, bevorzugt Amphotericin B, Fluconazol, Ketonazol, Miconazol und Griseofulvin, Caspofungin, Micafungin und/oder Anidulafungin, ist.

10. Zusammensetzung nach einem der vorhergehenden Ansprüche, wobei das mindestens eine Calcium-haltige Salz eine maximale Partikelgröße von 200 µm, bevorzugt von 100 µm, weiter bevorzugt von 64 µm aufweist.

11. Zusammensetzung nach einem der vorhergehenden Ansprüche, wobei die Zusammensetzung im Wesentlichen frei von Tensiden ist.

12. Verfahren zur Herstellung eines pharmazeutischen Wirkstoffträgers umfassend die Schritte
a. Bereitstellung einer Zusammensetzung nach einem der Ansprüche 1 bis 11,
b. Bereitstellen eines pharmazeutischen Wirkstoffs,
c. Mechanisches Vermischen der Zusammensetzung und des pharmazeutischen Wirkstoffs.

13. Verfahren nach Anspruch 12, wobei der pharmazeutische Wirkstoff ein partikulärer pharmazeutischer Wirkstoff ist.

14. Pharmazeutischer Wirkstoffträger hergestellt nach einem der Ansprüche 12 oder 13.

15. Verwendung einer Zusammensetzung nach einem der Ansprüche 1 bis 12 oder eines pharmazeutischen Wirkstoffträgers nach Anspruch 14 zur lokalen Freisetzung mindestens eines pharmazeutischen Wirkstoffs.
